(19) Europäisches Patentamt
European Patent Office
Office européen des brevets

(11) **EP 4 623 881 A1**

(12) **EUROPEAN PATENT APPLICATION**
published in accordance with Art. 153(4) EPC

(43) Date of publication:
**01.10.2025 Bulletin 2025/40**

(21) Application number: 22969578.8

(22) Date of filing: **28.12.2022**

(51) International Patent Classification (IPC):
***A61F 13/53*** *(2006.01)*

(52) Cooperative Patent Classification (CPC):
**A61F 13/53**

(86) International application number:
**PCT/CN2022/142842**

(87) International publication number:
**WO 2024/138426 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Foshan King Wonder Hi-tech Co., Ltd.**
**Foshan City, Guangdong Province 528100 (CN)**

(72) Inventors:
• **LIN, Yuwei**
  **Foshan, Guangdong 528100 (CN)**

• **LU, Xingwen**
  **Foshan, Guangdong 528100 (CN)**
• **XU, Yingbo**
  **Foshan, Guangdong 528100 (CN)**
• **YAN, ZhiJian**
  **Foshan, Guangdong 528100 (CN)**
• **ZENG, Donghui**
  **Foshan, Guangdong 528100 (CN)**
• **LU, Hongwei**
  **Foshan, Guangdong 528100 (CN)**

(74) Representative: **Canzler & Bergmeier**
**Patentanwälte**
**Partnerschaft mbB**
**Despag-Straße 6**
**85055 Ingolstadt (DE)**

(54) **ABSORBENT CORE BODY OF FLEXIBLE ELASTIC CORE**

(57)    An absorbent core body for rapidly infiltrating, flow guiding, absorbing, and preventing reverse osmosis. The absorbent core body utilizes flow guiding in a vertical direction and disordered isolation in a horizontal direction to prevent reverse osmosis. The absorbent core body comprises: at least one basic framework layer (1) formed by knitted yarns, the fibers of which are at least vertically distributed; at least one functional layer (3) which is located below the basic framework layer (1), is compounded by means of physical weaving or subjected to physical heat sealing, and absorbs and locks a liquid; and at least one anti-reverse osmosis functional layer (4) which is located above the basic framework layer (1), the fibers of which are irregularly distributed in the horizontal direction, and which is formed by anti-reverse osmosis yarns. The fibers of the basic framework layer (1) and the fibers of the anti-reverse osmosis functional layer (4) are both subjected to lyophilic treatment. The absorbent core body can be applied to personal and medical nursing products.

FIG.7

**Description**

**FIELD OF THE INENTION**

The present invention relates to an absorbent articles. More particularly, the present invention relates to an absorbent core body of rapid infiltration, guided absorption and preventable reverse osmosis, which makes use of diversion in the vertical direction and disorderly isolation in the horizontal direction to prevent reverse osmosis.

**BACKGROUND OF THE INVENTION**

Most of absorbent articles have a top sheet, an acquisition layer, a distribution layer and an absorbent core. The top sheet used is anti-reverse osmosis ne to fluid residue, and it is easy to result in fluid accumulation area between the top sheet and its lower layers with the absorbent core body being easy to form bulge, fault, slow infiltration, random fluid flow after imbibition.

In the prior art CN 202111009068.2 disclosed a production process of a composite core which comprised a bottom layer of an absorbent core, its middle layer and its top layer overlay from the bottom layer to the top layer. The trapezoidal structures were alternately set upper and lower during the production process of the middle layer of the absorbent core. The bottom layer, the middle layer and the top layer were compacted to form a thin composite core. When fluid entered the composite core, the first buffer space and the second buffer space were formed between the bottom layer and the top layer, and the trapezoidal structures in the middle layer of the absorbent core body were restored to store the fluid.

CN 108883019 B (E.G. Bianchi et al.) disclosed an absorbent article with channels constructed to increase absorbent efficiency which extended parallel to the longitudinal axis (80') and perpendicular to the longitudinal axis (80'). The absorbent article comprised a distribution layer (54) between a top sheet (24) and an absorbent core body (28) wherein the distribution layer further comprised a fibrous material, and a first and second longitudinally-extending channels (86a,b) substantially free of fibrous material to prove the efficiency of fluid absorbent.

CN 106943238 B (M.A. Robus et al.) disclosed an absorbent core body with an upper layer and a lower layer in which the upper layer comprised an upper surface and a lower surface opposite the upper surface and the lower layer comprised an upper surface and a lower surface opposite the upper surface. The upper layer comprised an open celled foam and the lower layer comprised a fibrous network wherein the upper layer comprises at least one aperture. The disclosed absorbent article had a horizontal center line. Its upper layer included a periphery within which two or more discontinuous parts were contained wherein the first discontinuous part had a first perimeter and the second discontinuous segment had a second perimeter in which the first discontinuous part and the second discontinuous part did not directly contact. The periphery of the upper layer of the absorbent core body contained a longitudinal axis and a central axis of a transverse axis with the central axis dividing the periphery of the upper layer of the absorbent core body into the first section and the second section. The lower layer of the absorbent core body included a longitudinal axis and a transverse axis with the transverse axis dividing the lower layer of the absorbent core body into the first section and the second section. The total surface area of the first section of the upper layer of the absorbent core body was at least 5% larger than the second section of the upper layer of the absorbent core. Therefor the upper layer in this patent had open celled foam to collect and absorb fluid, and the lower layer had dual absorbent core.

CN 107920938 B (A.V. Smith et al.) disclosed an absorbent article comprising a three-dimensional fluid permeable topsheet. The topsheet comprised a first layer comprising a hydrophobic material and a second layer comprising a hydrophilic material in which the first layer was bonded to the second layer. The topsheet comprised a plurality of recesses, a plurality of projections, and a plurality of land areas, wherein the land areas surrounded at least a majority of the plurality of projections and a plurality of the recesses for improving soft feeling and reducing exposure of skin to body fluid.

CN 202121304783.4 disclosed a high quality sanitary napkin in which reverse osmosis was prevented through the use of a reverse osmosis prevention area. When menstrual blood entered from the entrance and was absorbed by fluffy cotton, the middle of the lower end of the fluffy cotton was supported by protruding bubbles, so that menstrual blood would expand to the periphery of the fluffy cotton through gravity. When squeezed the middle area was supported by protruding bubbles so that the contraction range of the middle area was small and the majority of menstrual blood would not be squeezed out of the entrance so as to realize the function of reverse osmosis.

CN 201220646501.3 disclosed a fabric with a diversion function. The fabric body provided with a first diversion layer made from blend of ES fiber and fluffy fiber. With a double-layer diversion layer design, a large space could increase the capacity of temporary fluid storage, provide a better stress resistance and thus prevent reverse osmosis.

CN 201520239583.3 disclosed a surface fabric for panty liner or sanitary pad comprising a skin-friendly absorbent layer and a water-locked layer. The skin-friendly absorbent layer comprised a skin-friendly surface layer, a longitudinal diversion layer and a transverse diversion layer in which the skin-friendly surface layer was a surface layer made from disorderly combed hydrophilic fibers, the longitudinal diversion layer was a layer made from longitudinally combed hydrophilic fibers and connected to the bottom of the skin-friendly surface layer, and the transverse diversion layer was a layer made from transversely combed hydrophilic fibers and connected to the bottom of the longitudinal diversion layer. The water-locked layer was a layer made from hydrophobic fibers and comprised multiple through holes which were evenly distributed and in shape of funnel in order to form water-locked holes in which the wide ends of the water-locked holes was adjacent to the skin-friendly absorbent layer and the narrow ends of the water-locked holes was adjacent to an absorbent core. It was soft and skin-friendly with fluid being quickly absorbed and well dispersed, and it could effectively prevent reverse osmosis, keep the surface breathable and dry, and provide users comfortable and healthy use experience.

The above patents or patent applications in the art separately focused on the absorption rate, the cavity construction and the combination of hydrophilic and hydrophobic fibers in different layers in which the materials used were mainly short-staple fibers and nonwovens without forming a complete lamination design with excellent functions. Moreover, most of them realized local functions by increasing the absorption amount, designing the diversion grooves or channels, designing the multiple non-woven layers for diversion and reverse osmosis prevention, and breaking absorbent cores without forming an overall laminate scheme.

Therefore, it is necessary to provide a laminate which makes use of diversion in vertical direction and reverse osmosis prevention by disorderly isolation with rapid infiltration and diversion absorption to carry out rapid and continuous guidance of fluid so as to have a better absorption speed, a better strength after absorption, a less residual fluid accumulation, no surface seepage, and a more comfortable feeling to keep dry and soft.

## SUMMARY OF THE INVENTION

The object of the present invention is to provide an absorbent core body (a laminate) for rapid infiltration, guided absorption and preventable reverse osmosis, which makes use of diversion in vertical direction and disorderly isolation in horizontal direction to prevent reverse osmosis. The absorbent core body of the present invention has a better absorption speed, a better strength after absorption, a less residual fluid accumulation, no surface seepage, and a more comfortable feeling to keep dry and soft.

As in one aspect, in order to realize its object the present invention provides an absorbent core body for rapid infiltration, guided absorption and preventable reverse osmosis, in which the absorbent core body makes use of diversion in the vertical direction and disorderly isolation in the horizontal direction to prevent reverse osmosis, comprising:

at least one basic skeleton layer (also referred as Bsk layer hereafter) made from hosiery yarn with at least part of its fibers being vertically distributed fibers,
at least one functional layer (also referred as Func. Layer hereafter) for absorbing and locking fluid which is located below the basic skeleton layer and made by physically weaving, compounding or physically thermal bonding, and
at least one anti-reverse osmosis layer (also referred as anti-RO layer hereafter) which is located above the basic skeleton layer with its fibers irregularly distributed in the horizontal direction formed from anti-reverse osmosis yarn, wherein the fibers of the basic skeleton layer and the fibers of the anti-reverse osmosis layer have been hydrophilically(also referred as HPL hereafter) treated.

In the absorbent core body of the present invention, the basic skeleton layer can be a knitted structure of more than one sub-layer, preferably more than two sub-layers, and more preferably more than three sub-layers and less than nine sub-layers, and the fibers of each sub-layer can be arranged all transversely, all longitudinally, or crisscross.

In the absorbent core body of the present invention, in the sub-layers of the basic skeleton layer its even sub-layer(s) have vertical fibers and its odd sub-layer(s) are a laterally knitted fibers. Please note the even sub-layer(s) and the odd sub-layer(s) are just the result of counting ways for different sub-layers. The cross-section of vertical fibers can be either disorderly or in order with different shapes such as spiral coils, straight lines. The fibers can be interwoven longitudinally in multiple sub-layers for multiple times. They can also in a non-upright linear configuration, either by crossing multiple individual fibers or by crossing multiple clusters of fibers, with various interweaving methods available. The diameter of the vertical fibers can be controlled between 0.5D and 200D, preferably between 1D and 180D. The diameter of the horizontal fibers can be controlled between 1D and 200D, preferably between 10D and 180D. A three-sub-layer, five-sub-layer or nine-sub-layer structure in the vertical direction is particularly beneficial for instantaneous absorption and flow guidance to prevent fluid from accumulating.

In the absorbent core body of the present invention, the basic skeleton layer is based on knitting, which can be weft knitting, warp knitting, or a mixture of warp and weft knitting.

In the absorbent core body of the present invention, the fibers of the basic skeleton layer can be smooth or non-smooth, preferably velvet. The fibers can be coiled or straight, preferably coiled. The fibers can be sheath-core structure, hollow structure or blended structure, preferably hollow or blend structure, and more preferably hollow structure.

In the absorbent core body of the present invention, the used material in the basic skeleton layer and the top anti-reverse osmosis layer can be selected from the following group or any combination: TPE fiber, TPV fiber, cotton fiber, Tencel, Viscose fiber, bamboo fiber, polyacrylic acid fiber, polysulfone fiber, polyurea fiber, polyester (PES) and its derivatives fiber, nylon and its derivatives fiber, Spandex or Lycra and its derivatives fiber, polypropylene and its derivatives fiber, polyolefin and its derivatives fiber, aramide and its derivatives fiber, polyimide and its derivatives fiber, acrylic fiber, vinylon, polyvinyl chloride fiber, and Lyocell. Preferably, the material is non-hydrophilic material, such as polyester and its derivatives fiber, nylon and its derivatives fiber, Spandex or Lycra and its derivatives fiber, polypropylene and its derivatives fiber, polyolefin and its derivatives fiber, aramide and its derivatives fiber, polyimide and its derivatives fiber, and cotton fiber. More preferably, the material is fibers which are not expanded when absorbing. Most preferably, the material is fibers in which fluid can be absorbed but which can be dried fast.

In the absorbent core body of the present invention, the functional layer for absorbing and locking fluid is made from cellulose, natural compounds, synthetic polymer, etc. These materials can be either long or short fibers, which can be selected from any one, a combination of two or more, or a grafting of these materials in the following group consisting of: blended cotton, Tencel, viscose fiber, bamboo fiber, polyacrylic acid and its modified polymers, starch and its modified polymers, PVA and its modified polymers, acrylic fiber and its modified polymers, etc.

In the absorbent core body of the present invention, the functional layer for absorbing and locking fluid can be a single layer or a laminate with multiple layers.

In the absorbent core body of the present invention, the functional layer for absorbing and locking fluid can be formed by mechanical and physical methods such as knitting or weaving fibers onto the basic skeleton layer. Alternatively, it can be formed by bonding a powder, bead or villus with the ability to absorb and lock liquid onto the basic skeleton layer through an adhesive. Additionally, it can be a continuous non-woven state, preferably formed through processes such as hot extrusion, solution, hot press lamination, spunlace, hot rolling, hot melt spray, or electrospinning. In the absorbent core body of the present invention, the thickness of the functional layer for absorbing and locking fluid is kept within 3mm, preferably within 2mm, more preferably within 1.5mm. The volume for locking fluid is 2-50 times the total gram weight, preferably 5-30 times.

In the absorbent core body of the present invention, the functional layer for absorbing and locking fluid has also certain diffusion ability in its transverse directions. If the locking ability of the functional layer is too strong, it will lead to local thickening. Therefore, the functional layer for absorbing and locking fluid also has a rapid diffusion ability in its transverse directions within 3s, preferably within 2s, more preferably within 1s, in order to avoid local thickening.

In the absorbent core body of the present invention, at least one sub-layer which is disorderly or chaotic in its cross-section is constructed in the top anti-reverse osmosis layer and the at least one sub-layer is above the basic skeleton layer and extends to the top anti-reverse osmosis layer. It can also be more than two sub-layers and less than five sub-layers. The constructing method can be a physical method, such as sandpaper friction, snagging, needle hooking, electrostatic adsorption, roughening, polar fleece, fibers connecting, etc., or a chemical composite method, such as implantation, bonding, chemical infiltration, etc. After construction, its surface can be trimmed or not trimmed. No pruning is excellent for anti-reverse osmosis, but pruning is better than no pruning in terms of infiltration efficiency. The finer the fiber gap, the more disorderly the cross section, the more conducive to anti-reverse osmosis. If there are regular grooves or gaps between the fibers, it is more conducive to seepage or infiltration, and there is a certain sacrifice for anti-reverse osmosis.

In the absorbent core body of the present invention, the thickness of the top anti-reverse osmosis layer is maintained at 0.05mm to 3mm, preferably 0.08 to 2mm, more preferably 0.1mm to 1.8mm. The diameter of its fiber or its filament is 0.5D to 200D, preferably 0.5D to 180D, more preferably 0.5D to 150D.

In the absorbent core body of the present invention, the fluid leakage amount of the top anti-reverse osmosis layer is less than 1g at the pressure of 0.5KPa, preferably less than 0.6g, and more preferably less than 0.3g.

In the absorbent core body of the present invention, the top anti-reverse osmosis layer and/or the basic skeleton layer are treated for fluid absorbing and quick drying. Preferably, the both layers are treated. The hydrophilicity (also referred as HPLcity hereafter) of the top anti-reverse osmosis layer is at least twice, preferably three times, and more preferably four times, less than that of the basic skeleton layer. The material used for fluid absorbing and quick drying can be selected from one or any combination of the following group consisting of a mixture of water-dispersible polyester as its main component, hygroscopic silicone and its mixture, anionic polymer and its mixture, cationic polymer and its mixture, amphoteric polymer and its mixture, etc.

In the absorbent core body of the present invention, the top anti-reverse osmosis layer and the basic skeleton layer can be treated for fluid absorbing and quick drying first by surface spraying, impregnation or other method and then further by roller drying, oven drying, infrared drying, oven drum drying or other drying method.

In the absorbent core body of the present invention, the top anti-reverse osmosis layer has the smallest retention fluid area. The fluid retention area of the functional layer for absorbing and locking fluid can be same as that of the basic skeleton layer. Preferably the fluid retention area of the functional layer for absorbing and locking fluid is larger than that of the basic skeleton layer, i.e. the functional layer for absorbing and locking fluid has the largest fluid retention area. The retention fluid area of the functional layer for absorbing and locking fluid is more than 2 times that of the basic skeleton layer, preferably more than 8 times.

In the absorbent core body of the present invention, when the final laminate is tested by infiltration method the time spent by the fluid from the anti-reverse osmosis layer to the basic skeleton layer to the functional layer for absorbing and locking fluid is within 3s. When tested by reverse osmosis method the weight of the fluid kept in the anti-reverse osmosis layer is within 1.2g. When measured by the absorbed amount the weight of the anti-reverse osmosis layer after absorption is within 30 times the original weight before absorption. When the contacting area of the anti-reverse osmosis layer with body fluid is observed more than 20% of the contacting area contacted with blood at 37°C is returned to white within 3min and more than 80% of the contacting area contacted with urine is returned to white within 1min. When tested with a standard infiltration fluid, the areas with residual fluid of the basic skeleton layer and that of the functional layer for absorbing and locking fluid are more than twice that of the anti-reverse osmosis layer. When tested by climbing method, the climbing height is at least 2mm with stiffness 0.05Nm·cm or above. When its thickness is at least 1mm it is under the action of 5KPa pressure for 0.5s and then back to 0.5 KPa pressure the thickness is restored at least 60% in 1s. It is beneficial to be comfortable and insensitive. The more recovery, the stronger the insensitivity.

In the absorbent core body of the present invention, the thickness of the basic skeleton layer is 0.5mm-20mm, preferably 0.5-15 mm, more preferably 0.5-10mm. The thickness of the anti-reverse osmosis layer is 0.1mm -50mm, preferably 0.5-10mm, more preferably 0.5-6mm. The thickness of the functional layer for absorbing and locking fluid is 0.1mm-10mm, preferably 0.5-10mm, more preferably 0.5-3mm.

In the absorbent core body of the present invention, each layer can be cut separately without cutting off, or a combination of several layers can be cut without cutting off. Preferably the whole of all layers is cut, and the infiltration rate is increased by 0.5 to 5 times after cutting, or more preferably by 1 to 5 times.

As in another aspect, in order to realize its object the present invention further provides an absorbent core body for rapid infiltration, guided absorption and preventable reverse osmosis, in which the absorbent core body makes use of diversion in the vertical direction and disorderly isolation in the horizontal direction to prevent reverse osmosis, comprising:

at least one basic skeleton layer made from hosiery yarn with at least part of its fibers being vertically distributed fibers, and

at least one functional layer for absorbing and locking fluid which is located below the basic skeleton layer and made by physically weaving, compounding or physically thermal bonding,

wherein the basic skeleton layer has at least two layers of knitted structure, and in the sub-layers of the basic skeleton layer its even sub-layer(s) have vertical fibers and its odd sub-layer(s) are a laterally knitted fibers, and

wherein the fibers of the basic skeleton layer have been hydrophilically treated.

In the above absorbent core body of the present invention, the transversely woven fibers in the odd layer of the basic skeleton layer plays a role in preventing reverse osmosis to a certain extent.

As in still another aspect, in order to realize its object the present invention further provides an absorbent core body for rapid infiltration, guided absorption and preventable reverse osmosis, in which the absorbent core body makes use of diversion in the vertical direction and disorderly isolation in the horizontal direction to prevent reverse osmosis, comprising:

at least one basic skeleton layer made from hosiery yarn with at least part of its fibers being vertically distributed fibers, and

at least one functional layer for absorbing and locking fluid which is located below the basic skeleton layer and made by physically weaving, compounding or physically thermal bonding,

wherein the fibers in the basic skeleton layer have scales of more than 0.1mm or are velvet, and

wherein the fibers of the basic skeleton layer have been hydrophilically treated.

In the absorbent core body of the present invention, the irregular distribution of the velvet fibers of the basic skeleton layer in the horizontal direction also plays a role in preventing reverse osmosis to a certain extent.

As in still further another aspect, in order to realize its object the present invention further provides an application of the absorbent core body as described above in personal and healthy care articles.

The absorbent core body of the present invention utilizes the principle of fluid guidance by glass rod and the principle of polymer oriented stretching, along with the orderly orientation of fibers to guide and divert fluid, in which the fibers in the vertical direction can be straight, interlaced or disorderly only if the main fibers end or extend vertically. The fibers in the transversely section are disorderly in which a large number of tiny cavities are formed to prevent reverse osmosis. The finer the cavities, the more beneficial they are to prevent reverse osmosis.

The invention will be further explained in combination with the drawings and specific examples, but these specific examples are only explanations for some specific embodiments of the present invention, not a limitation on the present invention.

## BRIEF DESCRIPTION OF THE DRAWINGS

FIG. 1 is a product model diagram of an absorbent core body according to an embodiment of the present invention,

FIG. 2 is a a SEM image of the basic skeleton layer and the anti-reverse osmosis layer according to an embodiment of the present invention,

FIG. 3 is a typical surface view of the anti-reverse osmosis layer according to an embodiment of the present invention,

FIG. 4 is a typical structure diagram of the basic skeleton layer with a three-layer structure and a disorderly middle layer according to another embodiment of the present invention;

FIG. 5 is a diagram of the diversion diffusion effect of the basic skeleton layer with a three-layer structure according to an embodiment of the present invention,

FIG. 6 shows a display diagram of anti-reverse osmosis effects on the surface of the different anti-reverse osmosis layers according to embodiments of the present invention and in FIG. 6 DA means diffusion area,

FIG. 7 shows one of the interlaced intermediate fibers in the basic skeleton layer according to one embodiment of the present invention,

FIG. 8 shows a typical diagram of the knitting structure according to the embodiments of the present invention,

FIG. 9 is a display diagram for rapid fluid absorption, fluid diversion and anti-reverse osmosis according to one embodiment of the present invention, and

FIG. 10 is a display diagram of a dry and whitening surface of the anti-reverse osmosis layer according to one embodiment of the present invention.

In the above figures, the meaning of each numeral in the attached drawing is as follows:

1 the basic skeleton layer
3 the function layer for absorbing and locking fluid
4 the anti-reverse osmosis layer

## DESCRIPTION OF THE PREFERRED EMBODYMENT

In the following embodiment, the method for measuring thickness, gram weight, infiltration, reverse osmosis, absorption amount, number of standard fluid for infiltration, climbing, stiffness, absorption time, absorption ratio, diffusion area, etc. are as follows:

(1) Thickness Measuring:

A) Detection criteria: GB/T24218.2-2009

B) Detection device: two horizontal circular panels, consisting of a pres foot (upper circular plate) and a reference plate (lower circular plate). The press foot can be moved up and down and kept parallel to the reference plate with a surface area of 2,500 mm$^2$. The surface diameter of the reference plate is at least 50 mm larger than the press foot. The distance between the press foot and the reference plate can be shown in the measuring device with a scale interval of 0.01mm.

C) Measuring steps: cutting 10 samples each with an area of more than 2,500 mm$^2$, testing under the test standard atmosphere, using the device described in B), adjusting the load on the press foot to a uniform pressure of 0.5 kPa and adjusting the device to zero, raising the press foot and placing the sample on the reference plate without tension to ensure that the sample is positioned against the center of the press foot, lowering the press foot until contacting the sample for 10s, adjusting the device to measure the sample thickness and record the readings in mm, and repeating the above steps for the remaining 9 samples.

(2) Gram-weight Measuring:

A) Detection criteria: GB / T 24218.1-2009
B) Detection device:

Sample cutter, selected from the following instruments: 1, round cutter sampler with a sample area of at least 50,000mm2; 2, square mold with an area of at least 50,000mm2 (such as 250mm 200mm), and equipped with a cutter; 3, steel ruler with a grading value of 1mm and equipped with a cutter.

Balance, with error ranges between $\pm$ 0.1% of the measured mass.

C) Measuring steps: Cutting at least three testing samples from the sample with a round cutter sampler or with a square die and a cutter, each testing sample with at least 50,000mm². If the provided sample is not sufficient large to cut the testing sample with the specified size, then cut the rectangular sample with the largest size as possible and measure the area of the testing sample with a steel ruler. If the coefficient of variation is required, the number of testing samples is at least 5. The weight of each testing sample is measured with a balance under a standard atmosphere. The weight per unit area for each testing sample and their mean value in gram per square meter (g / m²) is calculated. If required, the coefficient of variation is calculated, expressed as a percentage.

(3) Infiltration Measuring:

A) Detection criteria: the company standard of the applicant

B) Sampling criteria: 5 testing samples in 125mmX125mm are taken for each sample.

C) Testing instruments and materials: 1. standard fluid for infiltration: sodium chloride solution in a concentration of 9 g/l with a surface tension of (70 $\pm$ 2) mN/m. 2. absorbent paper of 100mmX100mm with infiltration time less than (3 $\pm$ 0.5) s and fluid absorbent ratio (480 $\pm$ 30)%. 3. infiltration rate tester with a 50 ml burette, an electronic valve which can release 25ml in (3.5 $\pm$ 0.25)s, a 125mmX125mm transparent acrylic platform with a thickness of 5mm, and an acrylic press plate in thickness of 25mm and in weight of 500g with a hole in the middle in which an anti-corrosion timing electrode is installed.

D) Measuring steps: placing 5 layers of filter paper on the acrylic platform, placing the cut sample on the filter paper, aligning the burette and the acrylic press plate with a distance of 30mm between the burette and the testing sample, adding 5.0ml standard fluid for infiltration into the burette, turning on the electronic valve and starting to time when the standard fluid touches the surface of the testing sample and the electrode, stopping timing when the standard fluid is fully absorbed by the testing sample and the electrode can not detect any fluid left on the surface, and then recording the infiltration time.

(4) Anti-reverse Osmosis Measuring:

A) Detection criteria: the company standard of the applicant

B) Sampling criteria: 5 testing samples in 125mmX125mm are taken for each sample.

C) Testing instruments and materials: 1. standard fluid for infiltration: sodium chloride solution in a concentration of 9 g/l with a surface tension of (70 $\pm$ 2) mN/m. 2. absorbent paper of 100mmX100mm with infiltration time less than (3 $\pm$ 0.5) s and fluid absorbent ratio (480 $\pm$ 30)%. 3. infiltration rate tester with a 50 ml burette, an electronic valve which can release 25ml in (3.5 $\pm$ 0.25)s, a 125mmX125mm transparent acrylic platform with a thickness of 5mm, and an acrylic press plate in thickness of 25mm and in weight of 500g with a hole in the middle in which an anti-corrosion timing electrode is installed. 4. analog weights: 10cmX10cm with a total weight is 4000 $\pm$ 20g and a bottom with polyurethane rubber and PE film.

D) Measuring steps: placing 5 layers of filter paper on the acrylic platform, placing the cut sample on the filter paper, aligning the burette and the acrylic press plate with a distance of 30mm between the burette and the testing sample, adding 5.0ml standard fluid for infiltration into the burette, turning on the electronic valve and starting to time when the standard fluid touches the surface of the testing sample and the electrode, stopping timing when the standard fluid is fully absorbed by the testing sample and the electrode can not detect any fluid left on the surface, continuing to add the standard solution to the quantitative Qml in which Q is the filter paper weight multiplied by 3.30, removing the acrylic press plate and slowly placing the analog weight for 3 minutes to ensure that the fluid is evenly dispersed, weighing the weight of two filter paper as P1, placing the two layers of filter paper on the testing sample after slowly removing the analog weights, slowly placing the dried analog weights on the two layers of filter paper for 2 minutes, the reverse osmosis occurring at this point, removing the analog weights after 2 minutes, weighing the weight of the two layers of filter paper as P2, and counting the amount of reverse osmosis as WB = P 2-P1.

(5) Climbing Measuring:

A) Detection criteria: the company standard of the applicant

B) Sampling criteria: 5 testing samples in 30mmX250mm are taken for each sample.

C) Measuring steps: at a distance of 5$\pm$1 mm from the short and long sides drilling two holes with a diameter of (5 $\pm$1) mm from one short end of each testing sample, clamping the testing sample vertically on a horizontal bracket and keeping the holes at the bottom, making a glass rod passing through the two slots to maintain tension on the testing sample and keeping it vertical, placing the testing sample neatly and parallel to the measuring bar with protruding (15±2) mm below the zero point of the measuring bar, lowering the horizontal bracket until the zero of the measuring bar touches the fluid surface (the lower edge of the testing sample is below the surface (15 ± 2) mm), starting to time at this point, and recording the height of the fluid capillary rise after 10, 30, 60 seconds (recording can be made for 300 seconds if necessary). If the capillary rise is not a uniform line, then the highest point is recorded. The remaining testing samples are tested and the mean value is calculated.

(6) Stiffness Measuring:

A) Detection criteria: the company standard of the applicant
B) Sampling criteria: cutting 5 testing samples of (25 ± 1) mmX (250 ± 1) mm with long sides parallel to the test direction. The cutting shall be at least 50mm away from the sample edge.
C) Testing instrument: 40 ± 2mm wide, 200 ± 2mm long, at least 150mm high. Each side of the platform should be transparent, and the end of the platform is tilted downward and forms an angle of 41.5° with the horizontal direction.
D) Measuring steps: measuring the gram weight m of the testing sample, placing the instrument on a horizontal platform, placing the testing sample on the platform with one of its ends coinciding with the front edge of the platform, placing a steel ruler on the testing sample with the zero point of the ruler aligned with the edge mark D on the platform, pushing the ruler forward so that the testing sample protrudes to the front edge of the platform and bends downward under its own weight, moving the ruler forward at a constant speed (this can be achieved by using a device equipped with an electric drive) until the hanging end of the testing sample reaches the platform support surface, reading the overhang length of the testing sample on the scale of the steel ruler after 8 ± 2 seconds, the bending length being half the overhang length, and calculating the average bending length value (C) of the five testing samples. The stiffness (G) is: $G = m \times C^3 \times 10^{-3}$ *(mN.cm)*

(7) Diffusion area on the front and back:

A) Sampling criteria: testing samples in 100mmX100mm
B) Test instrument: 1. an iron supporting platform, 2. three iron rings, 3. an iron ruler of 15 cm with accuracy of 0.1cm, 4. a rigid cardboard / magnet (a circular hollow with a diameter of 6 cm in the middle), 5. two cameras of Logi C920 1080p, 6. a Studio (at least 40cm * 40cm * 40cm with LED light), 7. a pipette of 10ul, 8. standard solution with color and with viscosity of 10 ~ 12 cps / 30rpm, 9. a computer used for simultaneously connecting to two Logi cameras, 10. a Software of Image J, Logi Capture
C) Measuring steps: placing the testing sample on three iron rings on an iron supporting platform and placing an iron ruler next to it, placing two cameras 30cm above and below the testing sample with the lens parallel to the testing sample, setting the cameras to the same screen and same frequency and starting to video record, quickly adding 10 ul standard solution to the testing sample with a pipette, waiting for fluid being absorbed by the testing sample and 1.5 minutes thereafter stopping video recording, capturing an image when the testing sample contacted the solution for 1 minute, and calculating the diffusion areas of the testing sample on both its front and back sides by the Software and recording.

(8) Absorption Time Measuring:

A) Detection criteria: GB / T8939-2018
B) Sampling criteria: taking 5 pieces of testing samples for each sample.
C) Measuring steps: placing an absorption tester in a horizontal position, adding enough standard solution into the storage tank, starting the tester, clicking the washing/rinsing button twice, calibrating the added volume of fluid by the automatic adding device according to the instrument manual, removing the arc-shaped sample holder from the absorption rate tester and place it on a horizontal table, taking a piece of testing sample and removing its bottom release paper, gently sticking it to the arc-shaped testing area of the arc-shaped sample holder with the front end of the testing sample on the left side of the arc-shaped sample holder, the rear end on the right side and the center line aligned with the corresponding line of the outlet of the holder, then placing the arc-shaped sample holder attached with the testing sample in a fixed position of the absorption rate tester, entering the test interface of the absorption rate tester and setting the sample thickness to ensure that the standard test module falls freely to the surface of the testing sample, clicking the test button, adding (5.0±0.1) mL standard fluid into the standard test module by the automatic adding device, the timer being automatically started, and stopping timing automatically when the fluid surface disappears at the lowest point of the absorption zone.

(9) Absorption Rate Measuring:

A) Detection criteria: GB / T 8939-2018

B) Sampling criteria: taking 5 pieces of testing samples for each sample

C) Measuring steps: taking a testing sample, removing the release paper, weighing the weight (weight before absorption) with a balance with a sensitivity of 0.01g, holding one end of the testing sample with a clip in a way that the clip is perpendicular to the longitudinal direction of the testing sample without holding the built-in absorption layer,

immersing the testing sample together with the clip in distilled water or deionized water of (23±1°C) with the use surface of the testing sample up, gently pressing the testing sample until it is completely immersed for 60s, then lifting the clip to make the testing sample completely away from the water surface, hanging vertically for 90s, removing the clip, weighing the weight of the testing sample after absorption (weight after absorption), calculating the absorption amount based on the following formula, measuring 5 samples in the same steps, and taking the average value of 5 testing samples as the measurement result with rounding to one decimal place.

$$\text{Absorption Rate} = \frac{\text{weight after absorption} - \text{weight before absorption}}{\text{weight before absorption}}$$

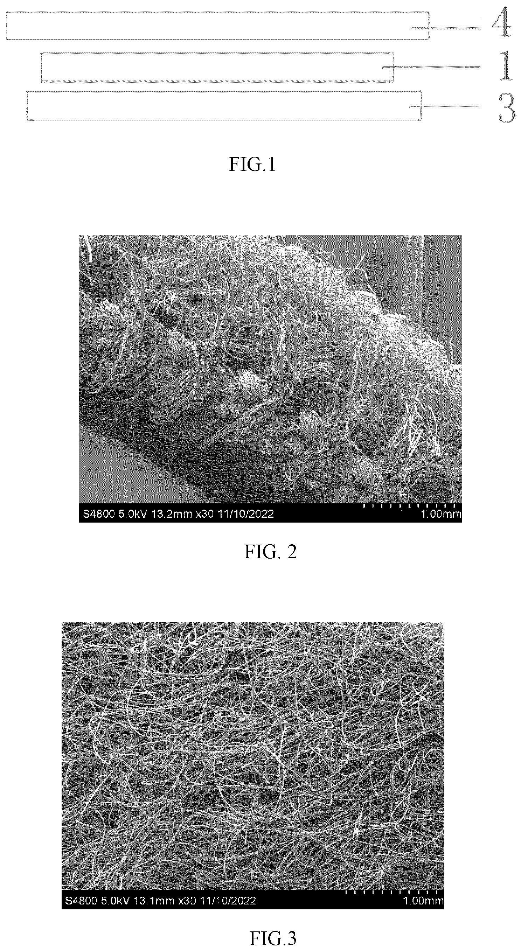

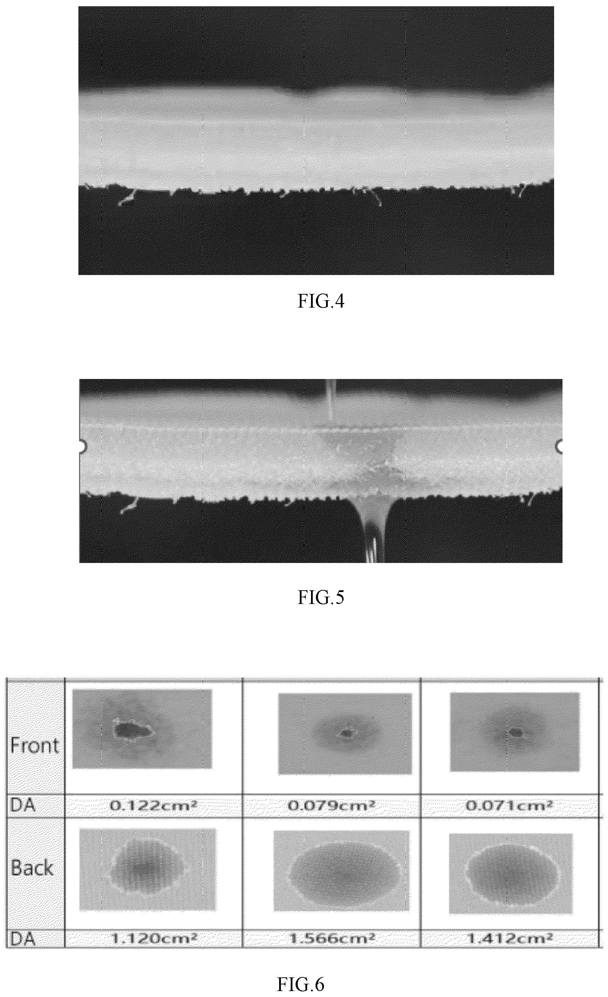

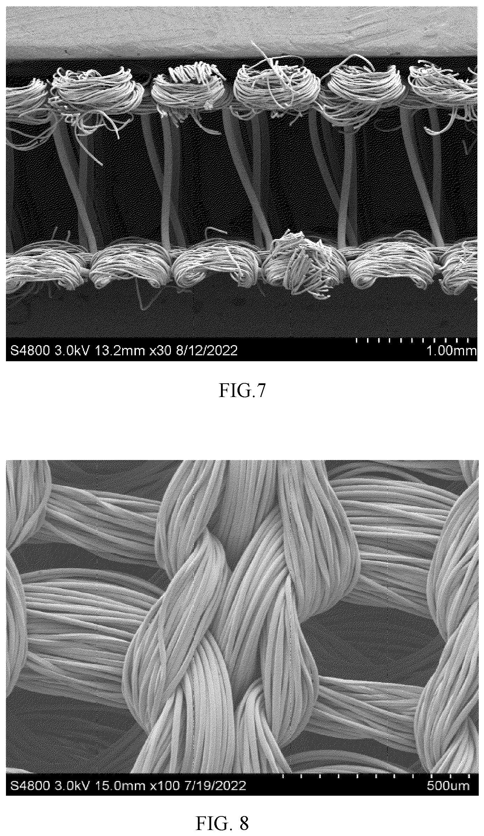

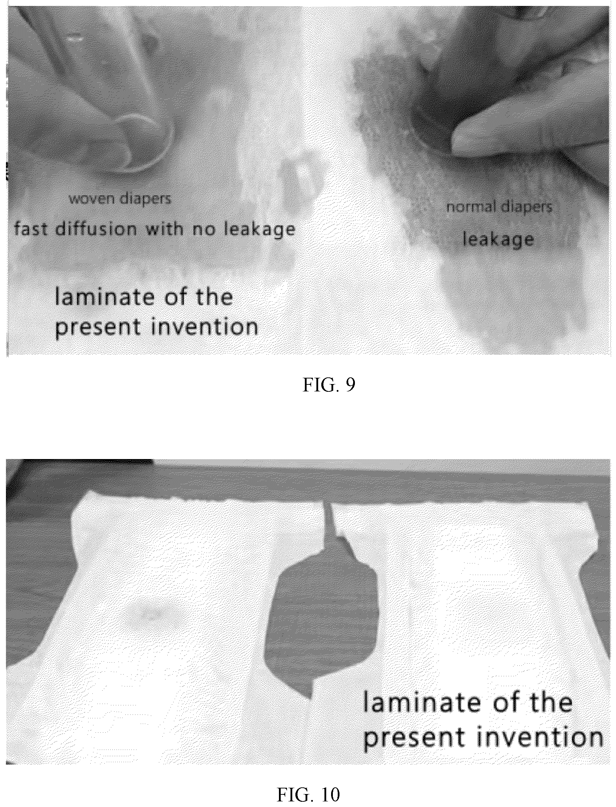

For simplicity, in the following examples (Examples 1-60), the respective experimental conditions and the respective experimental test structures are summarized in Table 1-Table 5. The drawings involved in the examples are further explained as follows: FIG. 1 shows a product model diagram of an absorbent core body (usually in form of a laminate) of the present invention, FIG. 2 is a SEM image (photo) of the basic skeleton layer and the anti-reverse osmosis layer in Example 2, FIG. 3 is a SEM image of the surface of the anti-reverse osmosis layer in Example 2, FIG. 4 shows a typical structure diagram of the basic skeleton layer with a three-layer structure and a disorderly vertical middle layer in Example 29, FIG. 5 shows a diversion diffusion effect diagram of the basic skeleton layer with a three-layer structure in Example 29, FIG. 6 shows a display diagram of anti-reverse osmosis effects with different surface settings of the anti-reverse osmosis layers in Example 1, 2 and 43, FIG. 7 shows the interlaced intermediate fibers in the basic skeleton layer in Example 2, FIG. 8 shows a typical diagram of the the knitting structure in the basic skeleton layers of all the examples, FIG. 9 is a display diagram for rapid fluid absorption, fluid diversion and anti-reverse osmosis in Example 2, and FIG. 10 is a display diagram with the surface of the anti-reverse osmosis layer in Example 2 becoming dry and whitened.

**Examples 1-9**

Table 1

| Ex. No | Ex.1 | Ex.2 | Ex.3 | Ex.4-6 | Ex.7-9 |
|---|---|---|---|---|---|
| experimental **objective** | Basic type | | | changing the basic skeleton layer with other materials | |
| **Bsk layer** | NHL PES material, mono-sub-layer structure | NHL PES, 3-sub-layer with the middle vertically disorderly | NHL PES, 3-sub-layer with the middle vertically interlaced | All replaced with hollow PES fibers | All replaced with PU |
| **connecting Bsk layer with Func. layer** | PO hot melt for spray method | | | same as Ex.1-3 | same as Ex.1-3 |
| **Func. layer** | AGMABs are wrapped with viscose spunlace with hydrophilic diffusion | | | same as Ex.1-3 | same as Ex.1-3 |
| **Anti-RO layer material** | NHL PES | same as 1 | same as 1 | same as Ex.1 | same as Ex.1 |
| **Anti-RO layer treatment** | 1-2mm in thickness with messy fluffy layer trimed and combed | | | same as Ex.1-3 | same as Ex.1-3 |

(continued)

| Ex. No | Ex.1 | Ex.2 | Ex.3 | Ex.4-6 | Ex.7-9 |
|---|---|---|---|---|---|
| experimental objective | Basic type | | | changing the basic skeleton layer with other materials | |
| Bsk layer | NHL PES material, mono-sub-layer structure | NHL PES, 3-sub-layer with the middle vertically disorderly | NHL PES, 3-sub-layer with the middle vertically interlaced | All replaced with hollow PES fibers | All replaced with PU |
| connecting Bsk layer & anti-RO layer | napping | | | same as Ex.1-3 | same as Ex.1-3 |
| Hygroscopic quick-dry treatment | Hygroscopic quick-dry treatment for hydrophilic PES mixture with anti-RO layer and Bsk layer treated in same proportion | | | same as Ex.1-3 | same as Ex.1-3 |
| thickness(um) | 1.5 | 2.5 | 2.4 | 1.5,2.5,2.4 | 1.5,2.5,2.4 |
| Infiltration (s) | 0.19 | 0.18 | 0.21 | 0.12,0.11,0.13 | 0.21,0.20,0.23 |
| RO (g) | 0.06 | 0.04 | 0.05 | 0.02,0.01,0.01 | 0.06,0.04,0.05 |
| Climbing (mm) | 64 | 63 | 65 | 67,67,66 | 64,63,65 |
| Stiffness (Nm.cm) | 1.7 | 2.5 | 2.0 | 1.8,2.3,1.9 | 1.0,1.5,1.2 |
| Absorption rate (times) | 23 | 23 | 23 | 23,23,23 | 23,23,23 |
| DA (front $mm^2$) | 0.130 | 0.080 | 0.073 | 0.102, 0.072, 0.065 | 0.135, 0.075, 0.072 |
| DA (back $mm^2$) | 1.220 | 1.623 | 1.426 | 1.130,1.524,1.35 | 1.142,1.583,1.45 |
| Effect | small stiffness, soft, thin, excellent function | larger stiffness, excellent anti-RO effect and faster inf. than Ex. 1, | less stiffness, and slightly inferior anti-RO effect and infiltration than Ex. 2 | better than Ex.2 on infiltration, anti-RO, and dry and whitened | very soft hand-feel |
| Note: AGMAB means acrylic grafted modified absorbent bead | | | | | |

**Examples 10-24**

**Table 2**

| Ex. No | Ex.10-12 | Ex.13-15 | Ex.16-18 | Ex.19-21 | Ex.22-24 |
|---|---|---|---|---|---|
| experimental objective | anti-RO layer is also hollow fiber | different fluid absorption material and structure | | different absorption materials, and structures and composite methods | |
| Bsk layer | same as Ex.4-6 | same as Ex. 1-3 | same as Ex.1-3 | same as Ex. 1-3 | same as Ex. 1-3 |
| connecting Bsk layer with Func. layer | same as 1-3 | same as 1-3 | same as Ex. 1-3 | knitted crochet | knitted crochet |

(continued)

| Ex. No | Ex.10-12 | Ex.13-15 | Ex.16-18 | Ex.19-21 | Ex.22-24 |
|---|---|---|---|---|---|
| Func. layer | same as 1-3 | AGMABs are wrapped with fluff pulp | A small amount of polypropylene is a skeleton to be bonded by fluff pulp | AGMABs are wrapped with Lyocell by spray adhesive bonding | AGMABs are wrapped with PVA by spray adhesive bonding |
| Anti-RO layer material | Hollow PES material | same as Ex.1 | same as Ex.1 | same as Ex.1 | same as Ex.1 |
| Anti-RO layer treatment | same as Ex. 1-3 | same as Ex. 1-3 | same as Ex.1-3 | same as Ex. 1-3 | same as Ex. 1-3 |
| connecting Bsk layer & anti-RO layer | same as Ex. 1-3 | same as Ex. 1-3 | same as Ex.1-3 | same as Ex.1-3 | same as Ex. 1-3 |
| hygroscopic quick-dry treatment | same as Ex. 1-3 | same as Ex. 1-3 | same as Ex.1-3 | same as Ex. 1-3 | same as Ex. 1-3 |
| Thickness (um) | 1.5,2.5,2.4 | 1.5,2.5,2.1 | 1.2,2.4,2.0 | 1.5,2.5,2.4 | 1.5,2.5,2.4 |
| Infiltration (s) | 0.09, 0.08, 0.10 | 0.21,0.19,0.2 3 | 0.22,0.20,0.25 | 0.18, 0.16, 0.20 | 0.17,0.16, 0.20 |
| RO (g) | 0.01, 0.01, 0.01 | 0.06, 0.08, 0.07 | 0.09, 0.11, 0.10 | 1.30, 0.32, 0.36 | 1.20, 0.30, 0.35 |
| Climbing (mm) | 69,68,69 | 63,64,63 | 43,44,45 | 71,69,72 | 71,70,71 |
| Stiffness (Nm.cm) | 1.8,2.3,1.9 | 2.1,4.9,4.5 | 2.2,3.2,3.0 | 1.6,2.1,1.8 | 2.0,2.5,2.1 |
| Absorption rate (times) | 23, 23, 23 | 13, 13, 13 | 10, 10, 10 | 15, 16, 15 | 17, 17, 17 |
| DA (front,mm$^2$) | 0.101,0.070,0 .063 | 1.2, 1.0, 1.11 | 1.4, 1.2, 1.32 | 1.2, 1.0, 1.11 | 1.2, 1.0, 1.11 |
| DA (back,mm$^2$) | 1.130,1.535,1 .378 | 1.16,1.85,1.4 4 | 1.35,1.80,1.56 | 1.18,1.89, 1.48 | 1.18, 1.89, 1.48 |
| Effect | Hollow fibers result in fast infil., less fluid accumul., and very good anti-RO. Diffusion to the bottom of Bsk layer is obvious, and the locking of absorbed fluid is clearly utilized. | Because the diffusion effect of the func. layer material is poor, the anti-RO effect becomes worse. | Because the diffusion effect of the func. layer material is poor, the absorbed amount drops obviously. | Absorption is obviously accelerated, the Bsk layer and the func. layer are increased via the fiber diversion principle as a glass rod, and anti-RO is slightly improved. | Absorption is obviously accelerated, the BSK layer and the func. layer are increased via the fiber diversion principle as a glass rod, and anti-RO is slightly improved. |

**Examples 25-33**

**Table 3**

| Ex. No | Ex.25-27 | Ex.28-30 | Ex.31-33 |
|---|---|---|---|
| experimental **objective** | No anti-RO layer | No anti-RO layer | No anti-RO layer |
| **Bsk layer** | same as Ex.1-3 | same as Ex.1-3 | same as Ex.1-3 but with fluff or scales more than 0.1mm |
| **connecting Bsk layer with Func. layer** | same as Ex.1-3 | same as Ex.1-3 | same as Ex.1-3 |
| **Func. layer** | same as Ex.1-3 | same as Ex.13-15 | same as Ex.1-3 |
| **Anti-RO layer material** | same as Ex.1 | same as Ex.13-15 | same as Ex.1 |
| **Anti-RO layer treatment** | no | no | no |
| **connecting Bsk layer & Anti-RO layer** | same as Ex.1-3 | same as Ex.1-3 | same as Ex.1-3 |
| **hygroscopic quick-dry treatment** | same as Ex.1-3 | same as Ex.1-3 | same as Ex.1-3 |
| **Thickness** (um) | 1.0,1.80,1.75 | 1.0,1.80,1.75 | 1.1,1.9,1.85 |
| **Infiltration** (s) | 0.21,0.19,0.23 | 0.21,0.19,0.23 | 0.19,0.17,0.21 |
| **RO** (g) | 1.50,0.40,0.48 | 1.50,0.40,0.48 | 1.02,0.66,0.72 |
| **Climbing** (mm) | 64,65,66 | 47,47,47 | 65,64,66 |
| **Stiffness** (Nm.cm) | 2.2,6.1,5.8 | 2.2,6.1,5.8 | 2.2,6.1,5.8 |
| **Absorption rate** (times) | 23,23,23 | 13,13,13 | 23,23,23 |
| **DA** (front, mm$^2$) | 1.25,0.99,1.08 | 1.2,1.0,1.11 | 1.25,0.99,1.08 |
| **DA** (back, mm$^2$) | 1.22,1.89,1.48 | 1.18,1.89,1.48 | 1.22,1.89,1.48 |
| **effect** | Ant-RO is very poor with the contrast of the DA on both sides reduced | Anti-RO is very poor with close DA on both sides | Anti-RO is poor but better than Ex. 25-27, the diversion is significantly increased, and the surface DA is obviously smaller than that of Bsk bottom |

**Example 34-42**

**Table 4**

| Example No | Ex.34-36 | Ex.37-39 | Ex.40-42 |
|---|---|---|---|
| experimental objective | Different construction methods of Anti-RO layer | | |
| Bsk layer | same as Ex.1-3 | same as Ex.1-3 | same as Ex.1-3 |
| connecting Bsk layer with Func. layer | same as Ex.1-3 | same as Ex.1-3 | same as Ex.1-3 |
| Func. layer | same as Ex.1-3 | same as Ex.1-3 | same as Ex.1-3 |
| Anti-RO layer material | same as Ex.1 | same as Ex.1 | same as Ex.1 |
| Anti-RO layer treatment | same as Ex.1-3 | same as Ex.1-3 | same as Ex.1-3 |
| connecting Bsk layer & Anti-RO layer | flocking | shaking grain | shaking grain with regular grooves |
| hygroscopic quick-dry treatment | same as Ex.1-3 | same as Ex.1-3 | same as Ex.1-3 |
| Thickness (um) | 1.2,2.4,2.0 | 1.5,2.5,2.4 | 1.5,2.5,2.4 |
| Infiltration (s) | 0.21,0.20,0.23 | 0.18,0.19,0.221 | 0.13,0.14,0.16 |
| RO (g) | 0.06,0.04,0.05 | 0.07,0.05,0.06 | 0.09,0.06,0.08 |
| Climbing (mm) | 64,63,65 | 64,63,65 | 64,63,65 |
| Stiffness (Nm.cm) | 1.8,2.3,1.9 | 1.7,2.2,1.7 | 1.2,1.8,1.5 |
| Absorption rate (times) | 23,23,23 | 23,23,23 | 23,23,23 |
| DA (front, mm$^2$) | 0.122,0.079,0.071 | 0.135,0.081,0.072 | 0.148,0.088,0.085 |
| DA (back, mm$^2$) | 1.120,1.566,1.412 | 1.126,1.675,1.535 | 1.156,1.823,1.624 |
| effect | same effect as that in Ex. 1-3 | same effect as that in Ex. 1-3, but Anti-RO effect slightly poorer than that in Ex. 1-3 | same effect as that in Ex. 34-36, but Anti-RO effect even poorer than that in Ex. 34-36 |

**Examples 46-60**

Table 5

| Ex. No | Ex.46-48 | Ex.49-51 | Ex.52-54 | Ex.55-57 | Ex.58-60 |
|---|---|---|---|---|---|
| experimental objective | hygroscopic quick-dry treatment with different levels for different layers | | | | |
| Bsk layer | same as Ex. 1-3 | same as Ex.1-3 | same as Ex.1-3 | same as Ex. 1-3 | same as Ex. 1-3 |
| connecting Bsk layer with Func. layer | same as Ex.3 | same as Ex.3 | same as Ex.3 | same as Ex.3 | same as Ex.3 |
| Func. layer | same as Ex.3 | same as Ex.3 | same as Ex.3 | same as Ex.3 | same as Ex.3 |
| Anti-RO layer material | same as Ex.1 | same as Ex.1 | same as Ex.1 | same as Ex.1 | same as Ex.1 |
| Anti-RO layer treatment | same as Ex.3 | same as Ex.3 | same as Ex.3 | same as Ex.3 | same as Ex.3 |
| connecting Bsk layer & Anti-RO layer | same as Ex.3 | same as Ex.3 | same as Ex.3 | same as Ex.3 | same as Ex.3 |
| hygroscopic quick-dry treatment | same agent as in Ex.1, no treatment for Anti-RO layer, and treatment for Bsk layer | same agent as in Ex.1, anti-RO layer and Bsk layer are treated for hydrophilicity, but degree of hydrophilicity of the former is twice lower than that of the latter | same agent as in Ex.1, anti-RO layer and Bsk layer are treated for hydrophilicity, but degree of hydrophilicity of the former is 5 times lower than that of the latter | same agent as in Ex.1, no treatment for Anti-RO layer, and treatment only for surface of Bsk layer toward Func. layer | same agent as in Ex.1, and no treatment neither for Anti-RO layer nor for Bsk layer |
| Thickness (um) | 1.5,2.5,2.4 | 1.5,2.5,2.4 | 1.5,2.5,2.4 | 1.5,2.5,2.4 | 1.5,2.5,2.4 |
| Infiltration (s) | 0.20,0.19, 0.23 | 0.18,0.17,0.20 | 0.18,0.16,0.19 | 0.24,0.25, 0.26 | 0.26,0.28, 0.30 |
| RO (g) | 0.02,0.02, 0.02 | 0.02,0.03,0.03 | 0.01,0.02,0.03 | 0.02,0.02, 0.04 | 0.02,0.01, 0.03 |
| Climbing (mm) | 60,62,63 | 63,64,65 | 60,62,63 | 60,62,63 | 58,60,62 |

EP 4 623 881 A1

14

(continued)

| Ex. No | Ex.46-48 | Ex.49-51 | Ex.52-54 | Ex.55-57 | Ex.58-60 |
|---|---|---|---|---|---|
| experimental **objective** | hygroscopic quick-dry treatment with different levels for different layers | | | | |
| **Bsk layer** | same as Ex. 1-3 | same as Ex.1-3 | same as Ex.1-3 | same as Ex. 1-3 | same as Ex. 1-3 |
| **Stiffness** (Nm.cm) | 1.7,2.5,2.0 | 1.7,2.5,2.0 | 1.7,2.5,2.0 | 1.7,2.5,2.0 | 1.7,2.5,2.0 |
| **Absorption rate** (times) | 23,23,23 | 23,23,23 | 23,23,23 | 23,23,23 | 23,23,23 |
| **DA** (front, $mm^2$) | 0.142,0.180, 0.165 | 0.115,0.075, 0.071 | 0.090,0.070, 0.062 | 0.142,0.180, 0.165 | 0.142,0.180, 0.165 |
| **DA** (back, $mm^2$) | 1.150,1.546, 1.358 | 1.172,1.638, 1.403 | 1.170,1.542, 1.302 | 1.172,1.553, 1.367 | 0.925,1.324, 1.125 |
| **effect** | **For Infiltration:** no treatment at all < treatment only for Bsk layer < no treatment for Anti-RO layer and only treatment for surface of Bsk layer toward Func. layer < anti-RO and Bsk layers treated for hydrophilicity with degree of hydrophilicity of the former 5 times lower than that of the latter < anti-RO and Bsk layers treated for hydrophilicity with degree of hydrophilicity of the former 2 times lower than that of the latter < full treatment; <br> **For Anti-RO effect:** opposite sequence as that of Infiltration; <br> **For ratio of front DA and back DA:** same sequence as that of Infiltration; <br> **Optimal embodiments:** degree of hydrophilicity of Anti-RO layer 5 times lower than that of Bsk layer. | | | | |

**Claims**

1. An absorbent core body for rapid infiltration, guided absorption and preventable reverse osmosis, in which said absorbent core body makes use of diversion in the vertical direction and disorderly isolation in the horizontal direction to prevent reverse osmosis, comprising:

   at least one basic skeleton layer made from hosiery yarn with at least part of its fibers being vertically distributed fibers,
   at least one functional layer for absorbing and locking fluid which is located below said basic skeleton layer and made by physically weaving, compounding or physically thermal bonding, and
   at least one anti-reverse osmosis layer which is located above said basic skeleton layer with its fibers irregularly distributed in the horizontal direction formed from anti-reverse osmosis yarn,
   wherein the fibers of said basic skeleton layer and the fibers of said anti-reverse osmosis layer have been hydrophilically treated.

2. The absorbent core body of claim 1, wherein said basic skeleton layer has sub-layers of knitted structure with the number of the sub-layers being more than two, preferably more than three and less than nine.

3. The absorbent core body of claim 2, wherein in the sub-sub-layers of said basic skeleton layer its even sub-layer(s) have vertical fibers and its odd sub-layer(s) are a laterally knitted fibers.

4. The absorbent core body of claim 1 wherein the hosiery yarn used in said basic skeleton layer is velvet yarn.

5. The absorbent core body of claim 1, wherein the hosiery yarn used in said basic skeleton layer is curled yarn.

6. The absorbent core body of claim 1, wherein the fibers of the hosiery yarn used in said basic skeleton layer have a hollow structure or a blended structure.

7. The absorbent core body of claim 1, wherein said functional layer for absorbing and locking fluid is formed by knitting or weaving the yarn for absorbing and locking fluid into said basic skeleton layer.

8. The absorbent core body of claim 1, wherein said functional layer for absorbing and locking fluid is formed by bonding a powder, bead or villus with the ability to absorb and lock liquid onto said basic skeleton layer through an adhesive.

9. The absorbent core body of claim 1, wherein said functional layer for absorbing and locking fluid is formed by hot extrusion, solution, hot press lamination, spunlace, hot rolling, hot melt spray, or electrospinning.

10. An absorbent core body for rapid infiltration, guided absorption and preventable reverse osmosis, in which said absorbent core body makes use of diversion in the vertical direction and disorderly isolation in the horizontal direction to prevent reverse osmosis, comprising:

    at least one basic skeleton layer made from hosiery yarn with at least part of its fibers being vertically distributed fibers, and
    at least one functional layer for absorbing and locking fluid which is located below said basic skeleton layer and made by physically weaving, compounding or physically thermal bonding,
    wherein said basic skeleton layer has more than two sub-layers of knitted structure, and in the sub-layers of said basic skeleton layer its even sub-layer(s) have vertical fibers and its odd sub-layer(s) are a laterally knitted fibers, and
    wherein the fibers of said basic skeleton layer have been hydrophilically treated.

11. An absorbent core body for rapid infiltration, guided absorption and preventable reverse osmosis, in which said absorbent core body makes use of diversion in the vertical direction and disorderly isolation in the horizontal direction to prevent reverse osmosis, comprising:

    at least one basic skeleton layer made from hosiery yarn with at least part of its fibers being vertically distributed fibers, and
    at least one functional layer for absorbing and locking fluid which is located below said basic skeleton layer and made by physically weaving, compounding or physically thermal bonding,

wherein the fibers in said basic skeleton layer have scales of more than 0.1mm or are velvet, and
wherein the fibers of said basic skeleton layer and the fibers of said anti-reverse osmosis layer have been hydrophilically treated.

12. An application of the absorbent core body as claimed in one of claims 1-11 in personal and healthy care articles.

FIG.1

FIG. 2

FIG.3

FIG.4

FIG.5

| | | | |
|---|---|---|---|
| Front | | | |
| DA | 0.122cm² | 0.079cm² | 0.071cm² |
| Back | | | |
| DA | 1.120cm² | 1.566cm² | 1.412cm² |

FIG.6

FIG.7

FIG. 8

FIG. 9

FIG. 10

## INTERNATIONAL SEARCH REPORT

| International application No. |
|---|
| **PCT/CN2022/142842** |

| A. | CLASSIFICATION OF SUBJECT MATTER |
|---|---|

A61F 13/53(2006.01)i

According to International Patent Classification (IPC) or to both national classification and IPC

| B. | FIELDS SEARCHED |
|---|---|

Minimum documentation searched (classification system followed by classification symbols)

IPC:A61F

Documentation searched other than minimum documentation to the extent that such documents are included in the fields searched

Electronic data base consulted during the international search (name of data base and, where practicable, search terms used)

CNABS; CNTXT; CNKI; VEN; WPABS; USTXT; WOTXT; EPTXT: 佛山金万达科技股份有限公司, 林裕卫, 陆星文, 许颖博, 颜志坚, 曾东辉, 陆鸿炜, 吸收芯, 导流, 反渗, 返渗, 竖直, 纵向, 水平, 横向, absorption, diversion, reverse osmosis, vertical, horizontal

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 217245223 U (FOSHAN RUIYUAN TECHNOLOGY CO., LTD.) 23 August 2022 (2022-08-23)<br>description, paragraphs [0005]-[0084], and figures 1 and 2 | 1-12 |
| Y | CN 107287768 A (TANG XINXIONG) 24 October 2017 (2017-10-24)<br>description, paragraphs [0004]-[0020], and figure 1 | 1-12 |
| A | CN 107468425 A (BARON (CHINA) CO., LTD.) 15 December 2017 (2017-12-15)<br>entire document | 1-12 |
| A | CN 1071072 A (PROCTER & GAMBLE CO.) 21 April 1993 (1993-04-21)<br>entire document | 1-12 |
| A | CN 215689124 U (GUANGDONG KAWADA SANITARY PRODUCTS CO., LTD.) 01 February 2022 (2022-02-01)<br>entire document | 1-12 |
| A | CN 204655299 U (SHANGHAI FENGGE NONWOVEN CO., LTD.) 23 September 2015 (2015-09-23)<br>entire document | 1-12 |

| ☑ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **26 June 2023** | **27 July 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2022/142842** |

| C. | DOCUMENTS CONSIDERED TO BE RELEVANT | |
| --- | --- | --- |
| Category* | Citation of document, with indication, where appropriate, of the relevant passages | Relevant to claim No. |
| A | CN 115337148 A (LUNALER HEALTH TECHNOLOGY CO., LTD.) 15 November 2022 (2022-11-15) entire document | 1-12 |
| A | WO 2021120988 A1 (FUJIAN HENGAN HOLDING CO., LTD.) 24 June 2021 (2021-06-24) entire document | 1-12 |

Form PCT/ISA/210 (second sheet) (July 2022)

**INTERNATIONAL SEARCH REPORT**
Information on patent family members

International application No.

**PCT/CN2022/142842**

| Patent document cited in search report | | | Publication date (day/month/year) | Patent family member(s) | | | Publication date (day/month/year) |
|---|---|---|---|---|---|---|---|
| CN | 217245223 | U | 23 August 2022 | None | | | |
| CN | 107287768 | A | 24 October 2017 | None | | | |
| CN | 107468425 | A | 15 December 2017 | None | | | |
| CN | 1071072 | A | 21 April 1993 | CN | 1130173 | C | 10 December 2003 |
| CN | 215689124 | U | 01 February 2022 | None | | | |
| CN | 204655299 | U | 23 September 2015 | None | | | |
| CN | 115337148 | A | 15 November 2022 | None | | | |
| WO | 2021120988 | A1 | 24 June 2021 | None | | | |

Form PCT/ISA/210 (patent family annex) (July 2022)

**REFERENCES CITED IN THE DESCRIPTION**

**Patent documents cited in the description**

- CN 202111009068
- CN 108883019 B, E.G. Bianchi
- CN 106943238 B, M.A. Robus
- CN 107920938 B, A.V. Smith
- CN 202121304783
- CN 201220646501
- CN 201520239583